# EUROPÄISCHE PATENTSCHRIFT

(11) **EP 0 657 407 B1**
(45) Veröffentlichungstag und Bekanntmachung des Hinweises auf die Patenterteilung: **10.12.1997**
(21) Anmeldenummer: 94118195.0
(22) Anmeldetag: 18.11.1994
(51) Int. Cl.: C07C 17/12, C07C 25/13

(54) **Verfahren zur Herstellung von 3-Fluor-4,6-dichlortoluol**
Process for the preparation of 3-fluoro-4,6-dichlorotoluene
Procédé pour la préparation de 3-fluoro-4,6-dichlorotoluène

(30) Priorität: 01.12.1993 DE 4340854
(43) Veröffentlichungstag der Anmeldung: 14.06.1995
(73) Patentinhaber: BAYER AG, 51368 Leverkusen (DE)
(72) Erfinder: Bussmann, Werner, Dr., D-51373 Leverkusen (DE)

(56) Entgegenhaltungen:
- EP-A- 0 114 604
- EP-A- 0 292 824
- US-A- 1 946 040

## Beschreibung

Die vorliegende Erfindung betrifft ein Verfahren zur Herstellung von 3-Fluor-4,6-dichlortoluol durch zweistufige selektive Chlorierung von 3-Fluortoluol, wobei ein verminderter Anteil des Nebenprodukts 3-Fluor-2,6-dichlortoluol erhalten wird.

3-Fluor-4,6-dichlortoluol ist ein Vorprodukt für die Herstellung von 2,4-Dichlorbenzoylchlorid, einem Schlüsselprodukt zur Herstellung von Antiinfektiva des Chinoloncarbonsäuretyps. Für die Herstellung dieser Verbindung sind eine Reihe von Synthesewegen beschrieben, bei denen jedoch entweder die Ausbeuten unbefriedigend sind oder mit technisch nur schwer handhabbaren Zwischenstufen und Produkten umgegangen werden muß.

Es ist bekannt, 3-Fluor-4,6-dichlortoluol aus 3-Amino-4,6-dichlortoluol herzustellen (siehe DE-A1-3 142 856). Dabei können zwar gute Ausbeuten realisiert werden, jedoch beinhaltet die Herstellung auf diesem Wege die Handhabung einer leicht zersetzlichen Triazenverbindung.

Eigene Versuche zur einstufigen Dichlorierung von 3-Fluortoluol in Gegenwart von Friedel-Crafts-Katalysatoren und Schwefel oder Thiazepinverbindungen haben ergeben, daß dabei Gemische von 3-Fluor-4,6-dichlortoluol und 3-Fluor-2,6-dichlortoluol erhalten werden, die maximal nur 66 Mol-% des 4,6-Isomers enthalten.

Es wurde nun ein Verfahren zur Herstellung von 3-Fluor-4,6-dichlortoluol mit reduzierter Bildung von 3-Fluor-2,6-dichlortoluol gefunden, das dadurch gekennzeichnet ist, daß man 3-Fluortoluol in Gegenwart von Friedel-Crafts-Katalysatoren und Schwefel zu einem 3-Fluor-6-chlortoluol und 3-Fluor-4-chlortoluol enthaltendem Gemisch chloriert und dieses in Gegenwart von Friedel-Crafts-Katalysatoren und Thiazepinverbindungen zu 3-Fluor-4,6-dichlortoluol chloriert.

Überraschenderweise ist die Selektivität der Bildung von 3-Fluor-4,6-dichlortoluol höher, wenn man statt in einem einstufigen Prozeß erfindungsgemäß zunächst mit einem Katalysatorsystem das Monochlorderivat herstellt, und dann mit einem anderen Katalysatorsystem zum Dichlorderivat weiterchloriert.

Als Friedel-Crafts-Katalysatoren für beide Reaktionsstufen des erfindungsgemäßen Verfahrens kommen z.B. die üblichen Metall- und Übergangsmetall-Halogenide in Frage. Bevorzugt sind Eisen(III)chlorid, Antimon(III)- und -(V)-chlorid und Aluminiumchlorid, besonders bevorzugt ist Eisen(III)chlorid. In die beiden Reaktionsstufen können gleiche oder verschiedene Friedel-Crafts-Katalysatoren eingesetzt werden. Vorzugsweise wendet man in beiden Reaktionsstufen die gleichen Friedel-Crafts-Katalysatoren an.

In die erste Reaktionsstufe kann man beispielsweise 0,05 bis 5 Gew.-% (bezogen auf 3-Fluortoluol) Friedel-Crafts-Katalysatoren einsetzen. Vorzugsweise beträgt diese Menge 0,1 bis 1 Gew.-%.

Schwefel kann z.B. in einer Menge von 0,05 bis 5 Gew.-% (bezogen auf 3-Fluortoluol) eingesetzt werden. Vorzugsweise liegt diese Menge bei 0,1 bis 1 Gew.-%. Der Schwefel kann in handelsüblicher pulveriger Form eingesetzt werden.

Man kann beide Reaktionsstufen des erfindungsgemäßen Verfahrens in Anwesenheit oder Abwesenheit von Lösungsmitteln durchführen. Als Lösungsmittel kommen z.B. halogenierte aliphatische Kohlenwasserstoffe in Frage, wie Tetrachlorkohlenstoff oder Dichlormethan sowie, insbesondere für die zweite Reaktionsstufe, auch längerkettige halogenierte aliphatische Kohlenwasserstoffe mit entsprechend höheren Siedepunkten. Vorzugsweise arbeitet man in beiden Reaktionsstufen ohne Zusätze von Lösungsmitteln.

Die erste Reaktionsstufe kann man beispielsweise bei 0 bis 60°C durchführen und dabei pro Mol 3-Fluortoluol z.B. 0,8 bis 1,1 Mol Chlor einleiten. Vorzugsweise arbeitet man bei 10 bis 30°C und mit 0,95 bis 1,01 Mol Chlor; besonders bevorzugt bei Raumtemperatur und mit einem Mol Chlor.

Nach Beendigung der ersten Reaktionsstufe des erfindungsgemäßen Verfahrens wird das Katalysatorsystem abgetrennt. Dies kann z.B. durch Dekantieren oder Filtrieren oder durch Abdestillation der Reaktionsprodukte geschehen. Das Katalysatorsystem kann bei einem weiteren Ansatz wieder verwendet werden.

Gegebenenfalls kann man vor oder nach der Abtrennung des Katalysatorsystems noch vorhandenen Chlorwasserstoff mit einem inerten Gas ausblasen, beispielsweise mit Stickstoff. Es ist vorteilhaft, vor der zweiten Reaktionsstufe, dort gegebenenfalls zu Störungen Anlaß gebende, leicht siedende Nebenprodukte, insbesondere flüchtige Schwefelverbindungen, abzutrennen. Wenn man die Reaktionsprodukte vom Katalysatorsystem durch Destillation abtrennt, kann man bei dieser Destillation einen Vorlauf abtrennen, der diese Nebenprodukte enthält. Sonst kann man z.B aus dem vom Katalysatorsystem befreiten Reaktionsgemisch einen kleinen Teil, beispielsweise 10 bis 20 Gew.-%, herausdestillieren. Die abgetrennten, leicht siedende Nebenprodukte bzw. eine sie enthaltende Fraktion kann man bei einem weiteren Ansatz wieder verwenden.

Das nach der ersten Reaktionsstufe vorliegende Reaktionsgemisch enthält im allgemeinen 3-Fluor-6-chlortoluol und 3-Fluor-4-chlortoluol im Gewichtsverhältnis 85-92:7-3. Bezogen auf eingesetztes 3-Fluortoluol liegen die Ausbeuten an diesem Gemisch im allgemeinen über 95 %. Häufig sind sie nahezu quantitativ.

Das vom Katalysatorsystem befreite und vorzugsweise wie oben beschrieben aufgearbeitete Reaktionsgemisch der ersten Reaktionsstufe wird dann der zweiten Reaktionsstufe zugeführt.

In die zweite Reaktionsstufe kann man beispielsweise 0,1 bis 10 Gew.-% (bezogen auf 3-Fluor-chlortoluole) Friedel-Crafts-Katalysatoren einsetzen. Vorzugsweise liegt diese Menge bei 0,2 bis 2,5 Gew.-%.

Als Thiazepinverbindungen kommen beispielsweise die in der EP-A1-292 824 (= US-Patent 4 851 596) beschriebenen in Frage. Vorzugsweise entsprechen sie einer der folgenden Formeln (I) bis (VII) 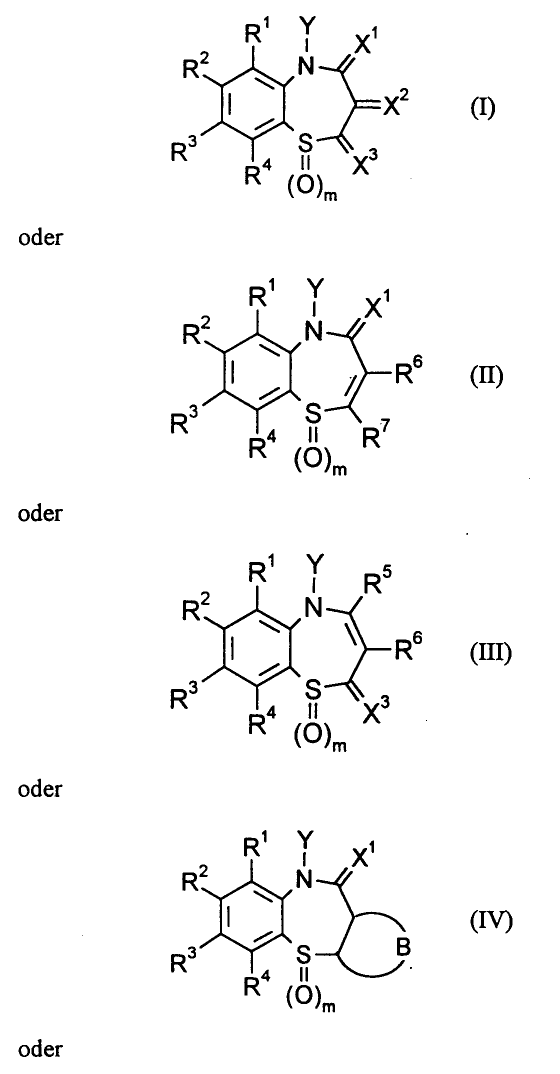 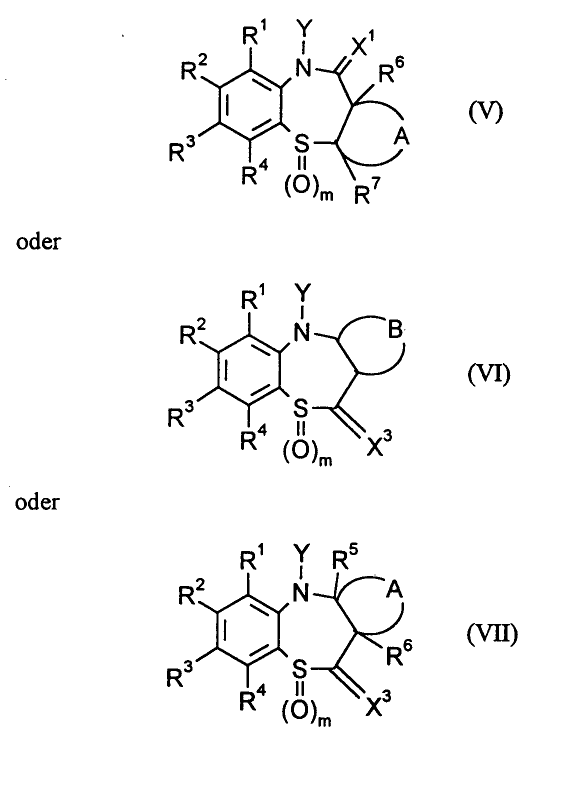 in denen
- R¹, R², R³, R⁴: gleich oder verschieden sind und für Wasserstoff, Hydroxy, Amino, Cyano, Halogen, Nitro, Nitroso, Sulfonyl, Sulfoxyl, Tosyl, Mercapto, Carboxyl, Carboxyamid, Carbalkoxy, Dithiocarboxyl, Thiocarboxylamid, Dithiocarbalkoxy, gegebenenfalls substituiertes Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino stehen oder untereinander einen oder mehrere gesättigte oder ungesättigte, gegebenenfalls substituierte isocyclische oder heterocyclische Kohlenstoffringe mit bis zu 8 C-Atomen bilden,
- Y: Wasserstoff, gegebenenfalls substituiertes Alkyl, Aryl, Heteroaryl, Acyl, Thioacyl, Acyloxy, Arylamino oder Acylamino bedeutet,
- X¹, X² und X³: unabhängig voneinander jeweils eine der folgenden Gruppierungen bedeuten: 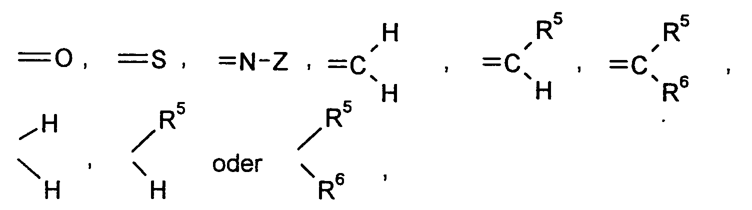
R⁵, R⁶ und R⁷ gleich oder verschieden sind und die Bedeutung von R¹ bis R⁴ besitzen, mit der Ausnahme, daß sie untereinander keinen cyclischen Ring bilden,
- Z: die Bedeutung von Y hat mit der Ausnahme, daß Z nicht gleich H ist,
- A: die Anellierung eines gegebenenfalls substituierten, gesättigten, isocyclischen oder heterocyclischen Rings mit bis zu 8 C-Atomen bedeutet,
- B: die Anellierung eines gegebenenfalls substituierten ungesättigten isocyclischen oder heterocyclischen Rings mit bis zu 8 C-Atomen bedeutet und
- m: 0 oder 1 bedeutet.

Als Substituenten der zuvorgenannten Reste kommen z.B. in Frage: Halogen, Nitro, Alkoxy, Alkyl, Aryl und Hetaryl, bevorzugt sind Halogen und Alkyl.

Als Alkylreste sind solche mit 1 bis 16 C-Atomen, insbesondere 1 bis 4 C-Atomen bevorzugt.

Als Arylreste sind solche mit 6 bis 10 C-Atomen, insbesondere 6 und 7 C-Atomen bevorzugt.

Als Heteroarylreste sind solche mit 4 bis 10 Atomen, davon 1 bis 3 Stickstoff-Sauerstoff- und/oder Schwefelatomen bevorzugt.

Als Alkoxyreste sind solche mit 1 bis 6 C-Atomen, insbesondere 1 bis 4 C-Atomen bevorzugt.

Als Aryloxyreste sind solche mit 6 bis 10 C-Atomen, insbesondere 6 und 7 C-Atomen bevorzugt.

Als Heteroaryloxyreste sind solche mit 4 bis 10 Atomen, davon 1 bis 2 Stickstoff-, Sauerstoff- und/oder Schwefelatomen bevorzugt.

Als Acyloxyreste sind solche mit 1 bis 7 C-Atomen, insbesondere 1 bis 4 C-Atomen bevorzugt.

Als Alkylthioreste sind solche mit 1 bis 6 C-Atomen, insbesondere 1 bis 4 C-Atomen bevorzugt.

Als Arylthioreste sind solche mit 4 bis 7 C-Atomen bevorzugt.

Als Heteroarylthioreste sind solche mit 4 bis 10 C-Atomen, davon 1 bis 2 Stickstoff-, Sauerstoff- und/oder Schwefelatomen bevorzugt.

Als Acylthio-, Acyl- und Thioacylreste sind jeweils solche mit 1 bis 7 C-Atomen bevorzugt.

Als Acylaminoreste sind solche mit 1 bis 8 C-Atomen bevorzugt.

Die Ringe A und B können unabhängig voneinander z.B. jeweils 1 bis 3 Stickstoff-, Sauerstoff- und/oder Schwefelatome enthalten.

Als Halogene sind Fluor, Chlor und Brom bevorzugt.

Ganz besonders bevorzugte Thiazepinverbindungen sind:
2,3-Dihydro-5H-benzo[b][1,4]thiazepin-4-on,
2-Methyl-2,3-dihydro-5H-benzo[b][1,4]thiazepin-4-on,
2-Ethyl-2,3-dihydro-5H-benzo[b][1,4]thiazepin-4-on,
2-Propyl-2,3-dihydro-5H-benzo[b][1,4]thiazepin-4-on,
3-Methyl-2,3-dihydro-5H-benzo[b][1,4]thiazepin-4-on,
2,3-Dimethyl-2,3-dihydro-5H-benzo[b][1,4]thiazepin-4-on,
5-Methyl-2,3-dihydro-5H-benzo[b][1,4]thiazepin-4-on,
5-Benzyl-2,3-dihydro-5H-benzo[b][1,4]thiazepin-4-on,
5-Acetyl-2,3-dihydro-5H-benzo[b][1,4]thiazepin-4-on,
5-Chlorocarbonyl-2,3-dihydro-5H-benzo[b][1,4]thiazepin-4-on,
2-Methyl-5-acetyl-2,3-dihydro-5H-benzo[b][1,4]thiazepin-4-on,
7-Chlor-2,3-dihydro-5H-benzo[b][1,4]thiazepin-4-on,
6,8-Dichlor-2,3-dihydro-5H-benzo[b][1,4]thiazepin-4-on,
6,8-Dichlor-2,3-dimethyl-2,3-dihydro-5H-benzo[b][1,4]thiazepin-4-on,
7-Methyl-2,3-dihydro-5H-benzo[b][1,4]thiazepin-4-on,
7-Trifluormethyl-2,3-dihydro-5H-benzo[b][1,4]thiazepin-4-on,
6,8-Dimethyl-2,3-dihydro-5H-benzo[b][1,4]thiazepin-4-on,
2,3-Dihydro-5H-[2,3]naphthaleno[b][1,4]thiazepin-4-on,
5H-Dibenzo[b,f][1,4]thiazepin-4-on,
kernchloriertes 5H-Dibenzo[b,f][1,4]thiazepin-4-on,
2,3-Tetramethylen-2,3-dihydro-5H-benzo[b][1,4]thiazepin-4-on,
6,8-Dimethyl-2,3-tetramethylen-2,3-dihydro-5H-benzo[b][1,4]thiazepin-4-on,
3H-5H-Benzo[b][1,4]thiazepin-2,4-dion,
3H-5H-Benzo[b][1,4]thiazepin-2-on-4-thion,
3H-5H-Benzo[b][1,4]thiazepin-2,4-dithion,
2,3-Dihydro-5H-benzo[b][1,4]thiazepin-4-thion,
2-Methyl-2,3-dihydro-5H-benzo[b][1,4]thiazepin-4-thion,
5-Acetyl-2,3-dihydro-5H-benzo[b][1,4]thiazepin-4-thion,
1-Oxo-2,3-dihydro-5H-1λ⁴-benzo[b][1,4]thiazepin-4-thion,
1-Oxo-7-chlor-5-methyl-2,3-dihydro-5H-1λ⁴-benzo[b][1,4]thiazepin-4-thion,
1-Oxo-2,3-dimethyl-2,3-dihydro-5H-1λ⁴-benzo[b][1,4]thiazepin-4-on,
3-Acetoxy-2,3-dihydro-5H-benzo[b][1,4]thiazepin-4-on,
3-Acetamino-2,5-dihydro-5H-benzo[b][1,4]thiazepin-4-on,
2,3,4,5-Tetrahydro-benzo[b][1,4]thiazepin,
2,3-Dimethyl-2,3,4,5-tetrahydro-benzo[b][1,4]thiazepin,
5-Ethyl-2,3,4,5-tetrahydro-benzo[b][1,4]thiazepin,
2-n-Tridecyl-2,3-dihydro-5H-benzo[b][1,4]thiazepin-4-on,
5-n-Pentyl-2,3-dihydro-5H-benzo[b][1,4]thiazepin-4-on,
8-Methoxy-2,3-dihydro-5H-benzo[b][1,4]thiazepin-4-on,
8-Ethoxy-2,3-dihydro-5H-benzo[b][1,4]thiazepin-4-on,
8-Methyl-2,3-dihydro-5H-benzo[b][1,4]thiazepin-4-on,
8-Chlor-6-methyl-2,3-dihydro-5H-benzo[b][1,4]thiazepin-4-on,
7,9-Dimethoxy-2,3-dihydro-5H-benzo[b][1,4]thiazepin-4-on,
7,9-Dimethyl-2,3-dihydro-5H-benzo[b][1,4]thiazepin-4-on,
7,8-Dimethyl-2,3-dihydro-5H-benzo[b][1,4]thiazepin-4-on,
7-Methoxy-2,3-dihydro-5H-benzo[b][1,4]thiazepin-4-on,
7,9-Dimethyl-2,3-tetramethylen-2,3-dihydro-5H-benzo[b][1,4]thiazepin-4-on,
2,3-Dihydro-5H-[1,2]naphthaleno[b][1,4]thiazepin-4-on,
2,3,7,9-Tetramethyl-2,3-dihydro-5H-benzo[b][1,4]thiazepin-4-on,

Man kann auch mehrere Thiazepinverbindungen gemeinsam einsetzen.

Thiazepinverbindungen können z.B. mit einer Menge von 0,02 bis 2 Gew.-% (bezogen auf 3-Fluor-chlortoluole) eingesetzt werden. Vorzugsweise liegt diese Menge bei 0,04 bis 1 Gew.-%.

Die zweite Reaktionsstufe kann man beispielsweise bei 50 bis 120°C durchführen und dabei pro Mol 3-Fluor-chlortoluole z.B. 0,8 bis 1,0 Mol Chlor einleiten. Vorzugsweise arbeitet man bei 60 bis 100°C und mit 0,95 bis 0,98 Mol Chlor.

Der Druck bei der Durchführung der ersten und zweiten Reaktionsstufe des erfindungsgemäßen Verfahrens kann beispielsweise im Bereich 1 bis 10 bar liegen. Bevorzugt ist Normaldruck.

Das elementare Chlor kann man in beide Reaktionsstufen gasförmig oder flüssig einleiten.

Nach Beendigung der zweiten Reaktionsstufe wird das Katalysatorsystem abgetrennt und gegebenenfalls vorhandener Chlorwasserstoff ausgeblasen, beispielsweise wie oben für die erste Reaktionsstufe beschrieben. Man kann den im allgemeinen weniger wertvollen Friedel-Crafts-Katalysator auch durch eine ein- oder mehrfache Wäsche mit Wasser entfernen und dann das verbleibende Reaktionsgemisch destillativ aufarbeiten. Dabei verbleiben die wertvollen Thiazepinverbindungen im Destillationsrückstand und können, gegebenenfalls zusammen mit dem Rückstand wieder eingesetzt werden.

Die Trennung der beiden im Reaktionsgemisch vorhandenen Isomeren (3-Fluor-4,6- und 3-Fluor-2,6-dichlortoluol) ist nach bekannten Methoden, z.B. durch Destillation möglich. Wenn man aus 3-Fluor-4,6-dichlortoluol 3-Fluor-4,6-dichlorbenzoylchlorid herstellen möchte, kann man die Isomerentrennung auch erst nach der Seitenkettenchlorierung aus dem Benzalchlorid/Benzotrichlorid-Gemisch durchführen.

Das erfindungsgemäße Verfahren zur Herstellung von 3-Fluor-4,6-dichlortoluol hat eine Reihe von Vorteilen. So kann es in üblichen technischen Anlagen und mit gängigen Chemikalien in beliebig großen Mengen 3-Fluor-4,6-dichlortoluol liefern und damit Vorprodukte für die Herstellung von Wirkstoffen aus der Gruppe der Chinoloncarbonsäuren günstig zur Verfügung stellen. Wirkstoffe aus der Gruppe der Chinoloncarbonsäsuren haben eine ständig zunehmende Bedeutung.

Es ist ausgesprochen überraschend, daß das erfindungsgemäße 2-Stufenverfahren Reaktionsgemische mit einem deutlich erhöhten Gehalt an 3-Fluor-4,6-dichlortoluol liefert.

### Beispiele

### Beispiel 1

110 g 3-Fluortoluol wurden in einem 4-Halskolben mit Rührer, Rückflußkühler, Thermometer und Gaseinleitungsrohr vorgelegt. Nach der Zugabe von 220 mg Eisen(III)chlorid und 110 mg Schwefel wurde bei 20°C (Kühlung mit einem Wasserbad) unter Rühren Chlor in einer Menge von 10 l/h eingeleitet. Nach 20 Stunden lagen im Reaktionsgemisch 91,4 Gew.-% 3-Fluor-6-chlortoluol und 5 Gew.-% 3-Fluor-4-chlortoluol vor. Das Reaktionsgemisch wurde einer Destillation unterworfen, wobei ein Vorlauf von 20 g abgenommen und in die nächste Partie zurückgeführt wurde. Der Hauptlauf (120 g) enthielt das katalysatorfreie 3-Fluor-chlortoluolgemisch. Der verbleibende Rückstand wurde ebenfalls wieder eingesetzt.

Das Beispiel wurde fünffach wiederholt, wobei sich dann schließlich eine Ausbeute von 98 % für das 3-Fluor-chlortoluol-Gemisch ergab.

### Beispiel 2

100 g des gemäß Beispiel 1 erhaltenen 3-Fluor-chlortoluol-Gemisches wurden in der gleichen Apparatur wie in Beispiel 1 beschrieben vorgelegt und 750 mg Eisen(III)chlorid und 150 mg 2,3-Dihydro-5H-benzo[b][1,4]thiazepin-4-on zugesetzt. Dann wurde mit einer Heizhaube auf 80°C erhitzt und in der oben beschriebenen Weise so lange Chlor eingeleitet, bis noch 5 Gew.-% des eingesetzten 3-Fluor-6-chlortoluols vorhanden waren. Das Reaktionsgemisch wurde zweimal mit Wasser (jeweils 20 ml) gewaschen und anschließend destilliert. Dabei wurde ein Vorlauf erhalten, der im wesentlichen unumgesetztes Ausgangsprodukt enthielt, das bei einem weiteren Ansatz wieder verwendet wurde. Danach wurden 100 g (= 90 % der Theorie) einer Mischung aus 76 Gew.-% 3-Fluor-4,6-dichlortoluol und 24 Gew.-% 3-Fluor-2,6-dichlortoluol abgetrennt. Der verbliebene Rückstand wurde bei einem weiteren Ansatz wieder verwendet.

Nach fünf Wiederholungen dieses Beispiels betrug die Ausbeute an 3-Fluor-4,6- und -2,6-dichlortoluol 98 % der Theorie.

### Beispiel 3 (Vergleichsbeispiel)

110 g 3-Fluortoluol wurden in der gleichen Apparatur wie in Beispiel 1 beschrieben vorgelegt. Nach Zugabe von 220 mg Eisen(III)-chlorid und 110 mg Schwefel wurde bei 20°C unter Rühren Chlor eingeleitet bis nur noch 1 Gew.-% des Monochlorderivates vorhanden war. Es wurde ein Gemisch mit 59 Gew.-% 4,6-Dichlor-3-fluortoluol und 31 Gew.-% 2,6-Dichlor-3-fluortoluol erhalten.

Bei der Destillation wurde ein Vorlauf von 20 g abgenommen, und dem nächsten Ansatz zugeführt. Als Hauptlauf wurden 130 g des Isomeren-Gemisches erhalten, der verbleibende Rückstand wurde ebenfalls wieder eingesetzt.

Nach fünffacher Wiederholung lag die Ausbeute bei 86 % der Theorie für das Gemisch.

### Beispiel 4 (Vergleichsbeispiel)

110 g 3-Fluortoluol wurden in der gleichen Apparatur wie in Beispiel 1 beschrieben vorgelegt. Nach Zugabe von 750 mg Eisen(III)-chlorid und 150 mg 2,3-Dihydro-5H-benzo[b][1,4]thiazepin-4-on wurde bei 20°C unter Rühren Chlor eingeleitet bis nur noch 1 Gew.-% des Monochlorderivates vorhanden war. Es wurde ein Gemisch mit je 43 Gew.-% 4,6-Dichlor-3-fluortoluol und 43 Gew.-% 2,6-Dichlor-3-fluortoluol erhalten.

Das Reaktionsgemisch wurde wie in Beispiel 2 beschrieben aufgearbeitet.

Bei der Destillation wurde ein Vorlauf von 20 g abgenommen, und dem nächsten Ansatz zugeführt. Als Hauptlauf wurden 120 g des Isomeren-Gemisches erhalten, der verbleibende Rückstand wurde ebenfalls wieder eingesetzt.

Nach fünffacher Wiederholung lag die Ausbeute bei 80 % der Theorie für das Gemisch.

## Patentansprüche

1. Verfahren zur Herstellung von 3-Fluor-4,6-dichlortoluol mit reduzierter Bildung von 3-Fluor-2,6-dichlortoluol, dadurch gekennzeichnet, daß man 3-Fluortoluol in Gegenwart von Friedel-Crafts-Katalysatoren und Schwefel zu einem 3-Fluor-6-chlortoluol und 3-Fluor-4-chlortoluol enthaltendem Gemisch chloriert und dieses in Gegenwart von Friedel-Crafts-Katalysatoren und Thiazepinverbindungen zu 3-Fluor-4,6-dichlortoluol chloriert.

2. Verfahren nach Anspruch 1, dadurch gekennzeichnet, daß man als Friedel-Crafts-Katalysatoren für beide Reaktionsstufen Eisen(III)chlorid, Antimon(III)chlorid, Antimon(V)chlorid oder Aluminiumchlorid einsetzt und zwar in die erste Reaktionsstufe 0,05 bis 5 Gew.-% (bezogen auf 3-Fluortoluol) und in die zweite Reaktionsstufe 0,1 bis 10 Gew.-% (bezogen auf 3-Fluor-chlortoluole).

3. Verfahren nach Ansprüchen 1 und 2, dadurch gekennzeichnet, daß man 0,05 bis 5 Gew.-% Schwefel (bezogen auf 3-Fluortoluol) einsetzt.

4. Verfahren nach Ansprüchen 1 bis 3, dadurch gekennzeichnet, daß man die erste Reaktionsstufe bei 0 bis 60°C durchführt und pro Mol 3-Fluortoluol 0,8 bis 1,1 Mol Chlor eingeleitet werden.

5. Verfahren nach Ansprüchen 1 bis 4, dadurch gekennzeichnet, daß man vor der zweiten Reaktionsstufe leicht siedende Nebenprodukte, insbesondere flüchtige Schwefelverbindungen, abtrennt.

6. Verfahren nach Ansprüchen 1 bis 5, dadurch gekennzeichnet, daß man als Thiazepinverbindungen solche der Formeln (I) bis (VII) 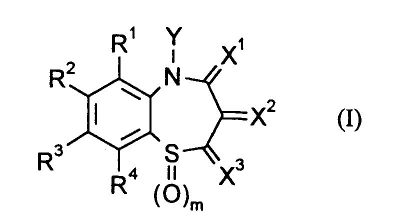 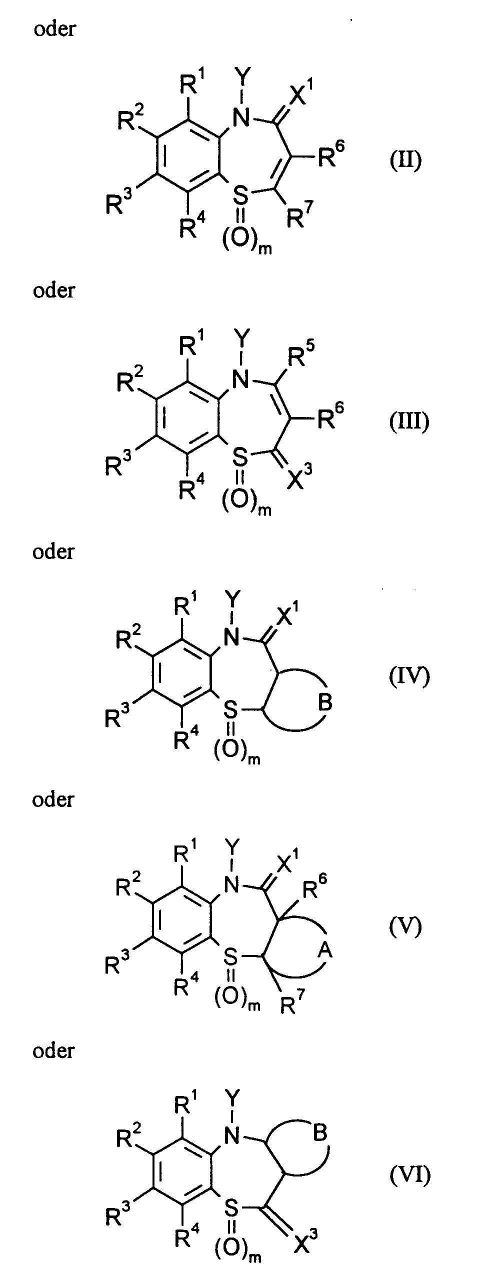 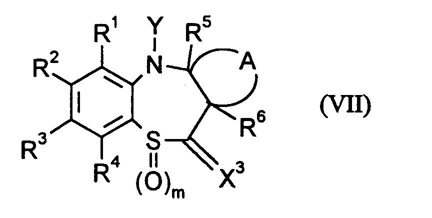 in denen
R¹, R², R³, R⁴ gleich oder verschieden sind und für Wasserstoff, Hydroxy, Amino, Cyano, Halogen, Nitro, Nitroso, Sulfonyl, Sulfoxyl, Tosyl, Mercapto, Carboxyl, Carboxyamid, Carbalkoxy, Dithiocarboxyl, Thiocarboxylamid, Dithiocarbalkoxy, gegebenenfalls substituiertes Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino stehen oder untereinander einen oder mehrere gesättigte oder ungesättigte, gegebenenfalls substituierte isocyclische oder heterocyclische Kohlenstoffringe mit bis zu 8 C-Atomen bilden,
Y Wasserstoff, gegebenenfalls substituiertes Alkyl, Aryl, Heteroaryl, Acyl, Thioacyl, Acyloxy, Arylamino oder Acylamino bedeutet,
X¹, X² und X³ unabhängig voneinander jeweils eine der folgenden Gruppierungen bedeuten: 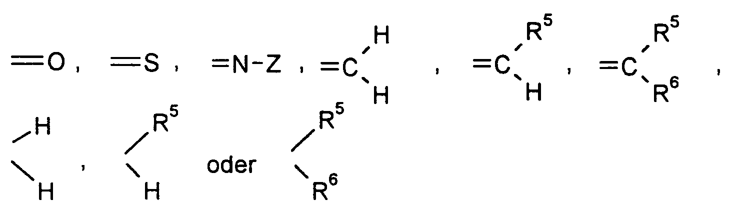
R⁵, R⁶ und R⁷ gleich oder verschieden sind und die Bedeutung von R¹ bis R⁴ besitzen, mit der Ausnahme, daß sie untereinander keinen cyclischen Ring bilden,
Z die Bedeutung von Y hat mit der Ausnahme, daß Z nicht gleich H ist,
A die Anellierung eines gegebenenfalls substituierten, gesättigten, isocyclischen oder heterocyclischen Rings mit bis zu 8 C-Atomen bedeutet,
B die Anellierung eines gegebenenfalls substituierten ungesättigten isocyclischen oder heterocyclischen Rings mit bis zu 8 C-Atomen bedeutet und
m 0 oder 1 bedeutet,
in einer Menge von 0,02 bis 2 Gew.-% (bezogen auf 3-Fluor-chlortoluole) einsetzt.

7. Verfahren nach Ansprüchen 1 bis 6, dadurch gekennzeichnet, daß man die zweite Reaktionsstufe bei 50 bis 120°C durchführt und pro Mol 3-Fluor-chlortoluole 0,8 bis 1,0 Mol Chlor eingeleitet werden.

8. Verfahren nach Ansprüchen 1 bis 7, dadurch gekennzeichnet, daß man nach Beendigung der zweiten Reaktionsstufe die Thiazepinverbindungen abtrennt und einen weiteren Ansatz wieder zufügt.

9. Verfahren nach Ansprüchen 1 bis 8, dadurch gekennzeichnet, daß man die nach der zweiten Reaktionsstufe im Reaktionsgemisch vorhandenen Isomeren 3-Fluor-4,6-dichlortoluol und 3-Fluor-2,6-dichlortoluol durch Destillation trennt.

10. Verfahren nach Ansprüchen 1 bis 8, dadurch gekennzeichnet, daß man die nach Beendigung der zweiten Reaktionsstufe vorliegenden 3-Fluor-dichlortoluol-Isomeren einer Seitenkettenchlorierung unterwirft und die Isomerentrennung aus dem Benzalchlorid/Benzotrichlorid-Gemisch durchführt.

## Claims

1. Process for preparing 3-fluoro-4,6-dichlorotoluene with reduced formation of 3-fluoro-2,6-dichlorotoluene, characterized in that 3-fluorotoluene is chlorinated in the presence of Friedel-Crafts catalysts and sulphur to give a mixture containing 3-fluoro-6-chlorotoluene and 3-fluoro-4-chlorotoluene and this is chlorinated in the presence of Friedel-Crafts catalysts and thiazepine compounds to give 3-fluoro-4,6-dichlorotoluene.

2. Process according to Claim 1, characterized in that the Friedel-Crafts catalysts used in both reaction stages are iron(III) chloride, antimony(III) chloride, antimony(V) chloride or aluminium chloride and in the first reaction stage are used in an amount of from 0.05 to 5% by weight (based on 3-fluorotoluene) and in the second reaction stage are used in an amount of from 0.1 to 10% by weight (based on 3-fluoro-chlorotoluenes).

3. Process according to either of Claims 1 and 2, characterized in that from 0.05 to 5% by weight of sulphur (based on 3-fluorotoluene) are used.

4. Process according to any of Claims 1 to 3, characterized in that the first reaction stage is carried out at from 0 to 60°C and from 0.8 to 1.1 mol of chlorine are introduced per mol of 3-fluorotoluene.

5. Process according to any of Claims 1 to 4, characterized in that low-boiling by-products, in particular volatile sulphur compounds, are separated off prior to the second reaction stage.

6. Process according to any of Claims 1 to 5, characterized in that the thiazepine compounds used are those of the formulae (I) to (VII) 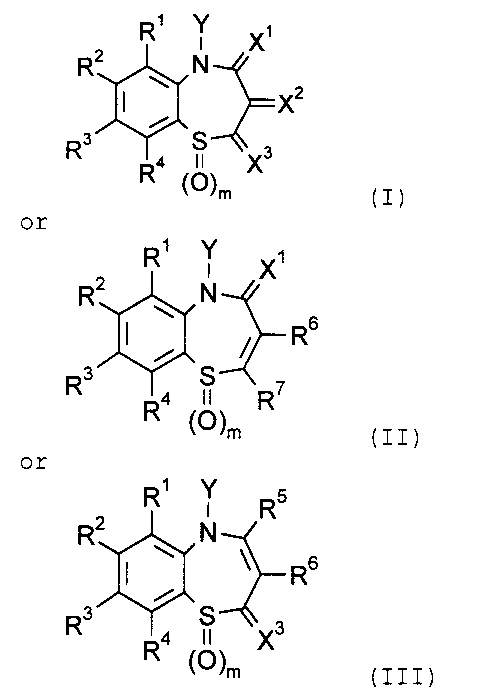 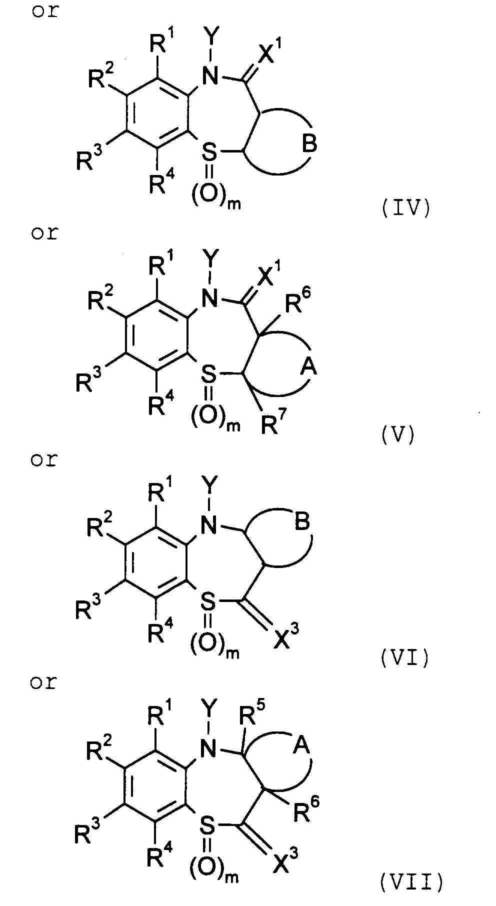 in which
R¹, R², R³, R⁴ are identical or different and represent hydrogen, hydroxy, amino, cyano, halogen, nitro, nitroso, sulphonyl, sulphoxyl, tosyl, mercapto, carboxyl, carboxyamide, carbalkoxy, dithiocarboxyl, thiocarboxylamide, dithiocarbalkoxy, optionally substituted alkyl, aryl, heteroaryl, alkoxy, aryloxy, heteroaryloxy, acyloxy, alkylthio, arylthio, heteroarylthio, acylthio, acyl, thioacyl or acylamino or among one another form one or more saturated or unsaturated, optionally substituted isocyclic or heterocyclic carbon rings having up to 8 carbon atoms,
Y is hydrogen, optionally substituted alkyl, aryl, heteroaryl, acyl, thioacyl, acyloxy, arylamino or acylamino,
X¹, X² and X³ are, independently of one another, each one of the following groups: 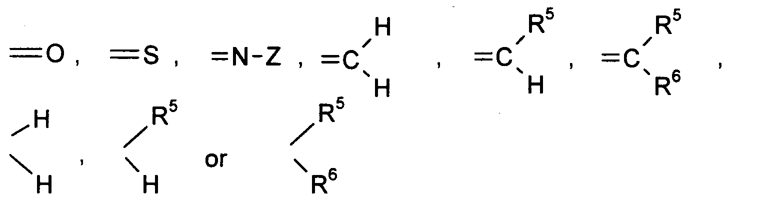
R⁵, R⁶ and R⁷ are identical or different and are as defined for R¹ to R⁴, with the exception that they do not form a cyclic ring among one another,
Z is as defined for Y, with the exception that Z is not H,
A represents the anellation of an optionally substituted, saturated, isocyclic or heterocyclic ring having up to 8 carbon atoms,
B represents the anellation of an optionally substituted, unsaturated, isocyclic or heterocyclic ring having up to 8 carbon atoms, and
m is 0 or 1,
and are used in an amount of from 0.02 to 2% by weight (based on 3-fluoro-chlorotoluenes).

7. Process according to any of Claims 1 to 6, characterized in that the second reaction stage is carried out at from 50 to 120°C and from 0.8 to 1.0 mol of chlorine is introduced per mol of 3-fluoro-chlorotoluenes.

8. Process according to any of Claims 1 to 7, characterized in that after completion of the second reaction stage the thiazepine compounds are separated off and again added to a further batch.

9. Process according to any of Claims 1 to 8, characterized in that the isomeric 3-fluoro-4,6-dichlorotoluene and 3-fluoro-2,6-dichlorotoluene present in the reaction mixture after the second reaction stage are separated by distillation.

10. Process according to any of Claims 1 to 8, characterized in that the 3-fluoro-dichlorotoluene isomers present after completion of the second reaction stage are subjected to a side-chain chlorination and the isomer separation is carried out from the benzal chloride/benzotrichloride mixture.

## Revendications

1. Procédé de préparation de 3-fluoro-4,6-dichlorotoluène avec formation réduite de 3-fluoro-2,6-dichlorotoluène, caractérisé en ce qu'on chlore du 3-fluorotoluène en présence de catalyseurs Friedel-Crafts et de soufre pour donner un mélange contenant du 3-fluoro-6-chlorotoluène et du 3-fluoro-4-chlorotoluène et on chlore ce mélange en présence de catalyseurs de Friedel-Crafts et de composés de thiazépine en 3-fluoro-4,6-dichlorotoluène.

2. Procédé selon la revendication 1, caractérisé en ce qu'on utilise comme catalyseurs Friedel-Crafts pour les deux étapes de réaction du chlorure de fer (III), du chlorure d'antimoine (III), du chlorure d'antimoine (V) ou du chlorure d'aluminium et cela à la première étape de réaction à une concentration de 0,05 à 5 % en poids (par rapport au 3-fluorotoluène) et à la deuxième étape de réaction en une concentration de 0,1 à 10 % en poids (par rapport aux 3-fluorochlorotoluènes).

3. Procédé selon les revendications 1 et 2 caractérisé en ce qu'on utilise 0,05 à 5 % en poids de soufre (par rapport au 3-fluorotoluène).

4. Procédé selon les revendications 1 à 3, caractérisé en ce qu'on réalise la première étape de réaction entre 0 et 60°C et qu'on introduit par mole de 3-fluorotoluène 0,8 à 1,1 mol de chlore.

5. Procédé selon les revendications 1 à 4, caractérisé en ce qu'on sépare avant la deuxième étape de réaction des sous-produits volatils, notamment des composés du soufre volatils.

6. Procédé selon les revendications 1 à 5, caractérisé en ce qu'on utilise comme composés de thiazépine ceux des formules (I) à (VII) 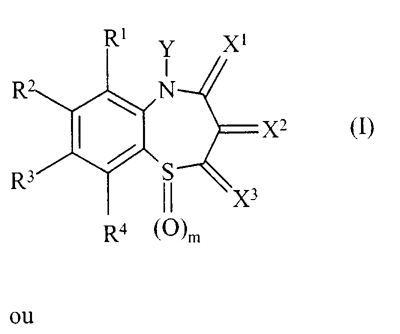 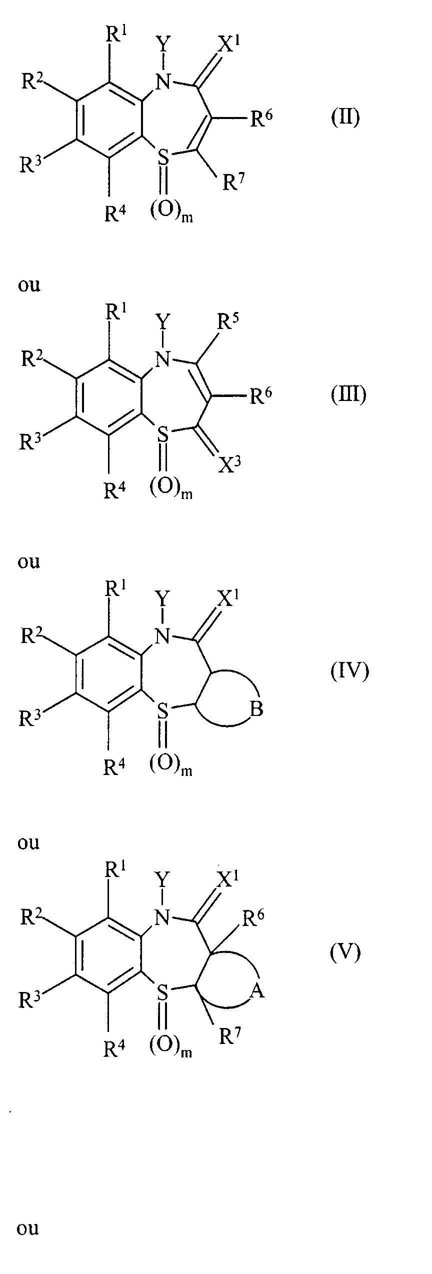 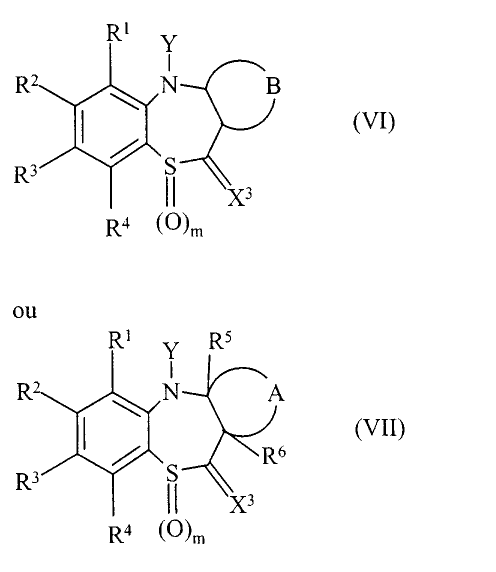 où
R¹, R², R³, R⁴ sont identiques ou différents et représentent les hydrogène, hydroxy, amino, cyano, halogène, nitro nitroso, sulfonyle, sulfoxyle, tosyle, mercapto, carboxyle, carboxyamide, carbalcoxy, dithiocarboxyle, thiocarboxylamide, dithiocarbalcoxy, alkyle substitué le cas échéant, aryle, hétéroaryle, alcoxy, aryloxy, hétéroaryloxy, acyloxy, alkylthio, arylthio, hétéroarylthio, acylthio, acyle, thioacyle ou acylamino ou forment entre eux un ou plusieurs cycles carbonés isocycliques ou hétérocycliques saturés ou insaturés, le cas échéant substitués avec jusqu'à 8 atomes de C,
Y représente l'hydrogène les groupes éventuellement substitués alkyle, aryle, hétéroaryle, acyle, thioacyle, acyloxy, arylamino ou acylamino,
X¹, X² et X³ représentent indépendamment l'un de l'autre respectivement l'un des groupes suivants : 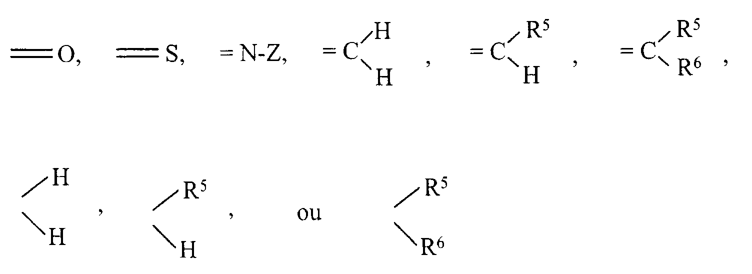
R⁵, R⁶ et R⁷ sont identiques ou différents et ont la signification de R¹ à R⁴, à l'exception qu'ils ne forment pas entre eux de cycles,
Z a la signification de Y à l'exception que Z n'est pas H,
A représente un cycle condensé isocyclique ou hétérocyclique saturé substitué le cas échéant pouvant avoir jusqu'à 8 atomes de C,
B représente un cycle condensé isocyclique ou hétérocyclique insaturé substitué le cas échéant avec jusqu'à 8 atomes de C et
m représente 0 ou 1,
en une quantité de 0,02 à 2 % en poids (par rapport aux 3-fluorochlorotoluènes).

7. Procédé selon les revendications 1 à 6, caractérisé en ce qu'on réalise la deuxième étape de réaction entre 50 et 120°C et qu'on introduit par mole de 3-fluorochlorotoluènes 0,8 à 1,0 mol de chlore.

8. Procédé selon les revendications 1 à 7, caractérisé en ce qu'après la fin de la deuxième étape de réaction on sépare les composés de thiazépine et on les réutilise à un autre lot de fabrication.

9. Procédé selon les revendications 1 à 8, caractérisé en ce qu'on sépare les isomères présents dans le mélange réactionnel après la deuxième étape de réaction, le 3-fluoro-4,6-dichlorotoluène et le 3-fluoro-2,6-dichlorotoluène par distillation.

10. Procédé selon les revendications 1 à 8 caractérisé en ce qu'à la fin de la deuxième étape de réaction on soumet les isomères de 3-fluorodichlorotoluène à une chloration de chaîne latérale et on réalise la séparation des isomères du mélange chlorure de benzylidène/benzotrichlorure.
